# EUROPÄISCHE PATENTANMELDUNG

(11) **EP 1 918 718 A1**
(43) Veröffentlichungstag der Anmeldung: **07.05.2008**
(21) Anmeldenummer: 06123234.4
(22) Anmeldetag: 31.10.2006
(51) Int. Cl.: G01N 33/86, C12Q 1/00, C12Q 1/56

(54) **Verfahren und Vorrichtungen zur elektrochemischen Bestimmung von Faktor Xa-Inhibitoren in Blutproben**

(71) Anmelder: Roche Diagnostics GmbH, 68305 Mannheim (DE); F. Hoffmann-la Roche AG, 4070 Basle (CH)
(72) Erfinder: Die Erfindernennung liegt noch nicht vor
(74) Vertreter: Silber, Anton

(57) **Zusammenfassung**

Die Erfindung betrifft Verfahren und Vorrichtungen, insbesondere Testelemente wie Teststreifen, zur Bestimmung von Faktor Xa-Inhibitoren, insbesondere von Heparinen und fraktionierten oder niedermolekularen Heparinen sowie direkten FXa-Inhibitoren, in Blutproben, welche dadurch gekennzeichnet sind, dass in einem ersten Schritt eine Probe des zu untersuchenden Blutes mit einem Nachweisreagenz, welches zumindest ein Thrombinsubstrat, welches aus einem Peptidrest besteht, der durch Thrombin abgespalten werden kann und der über das Carboxylende amidisch an eine elektrogene Substanz gebunden ist, enthält, und mit einer bekannten Menge von Faktor Xa in Kontakt gebracht wird, anschließend in einem zweiten Schritt die Menge oder Aktivität der elektrogenen Substanz, welche durch die Faktor Xa-vermittelte Thrombinaktivierung vom Thrombinsubstrat abgespalten wird, und/oder deren zeitlicher Verlauf als Messsignal mit elektrochemischen Methoden bestimmt wird, und schließlich in einem dritten Schritt anhand dieses Messsignals die Menge des Faktor Xa-Inhibitors in der Probe des zu untersuchenden Blutes oder eine damit korrelierende Messgröße, insbesondere eine damit korrelierende Gerinnungszeit, ermittelt wird.

## Beschreibung

### Gebiet der Erfindung:

Die Erfindung betrifft Verfahren zur elektrochemischen Bestimmung von Faktor Xa-Inhibitoren, insbesondere von Heparinen und Heparinderivaten sowie direkten Faktor Xa-Inhibitoren, in Blutproben.

Weiterhin betrifft die Erfindung Testelemente auf trockenchemischer Basis und Testelement-Analysesysteme zur elektrochemischen Bestimmung von Faktor Xa-Inhibitoren, insbesondere von Heparinen und Heparinderivaten sowie direkten Faktor Xa-Inhibitoren, in Blutproben.

### Stand der Technik:

Zur Prophylaxe und Therapie von hämostatischen Störungen (Störungen des Gerinnungssystems) werden in der klinischen Praxis oft Antikoagulantien eingesetzt, wozu insbesondere auch Heparine zählen. Heparine und Heparinderivate werden insbesondere zur Therapie und Prophylaxe von thromboembolischen Erkrankungen verwendet. Sie sind sehr effektiv bei der Prophylaxe und Behandlung von Beinvenenthrombosen, Lungenembolien und zur Behandlung von instabiler Angina pectoris und akutem Myokardinfarkt. Vielfachen Einsatz finden sie auch während Operationen, insbesondere bei kardiologischen Eingriffen (Bypass) und bei Bluttransfusionen.

Die Wirkung der Heparine beruht hauptsächlich auf ihrer Interaktion mit Antithrombin III (ATIII), wodurch sie eine Konformationsänderung des ATIII bewirken. Hierdurch wird die Inaktivierung bestimmter Gerinnungsenzyme (Thrombin (FIIa), Faktor Xa (FXa) und Faktor IXa) beschleunigt und somit die Gerinnung verzögert. Heparine können somit in die Klasse der Faktor Xa-Inhibitoren eingeordnet werden. Weitere Faktor Xa-Inhibitoren sind beispielsweise bestimmte Oligosaccharide wie das Pentasaccharid Fondaparinux oder sich noch in der klinischen Entwicklung befindliche niedermolekulare direkte Faktor Xa-Inhibitoren, welche verschiedenen Substanzklassen zugeordnet werden können. Neben den seit langer Zeit eingesetzten unfraktionierten Heparinen (UFH=unfractioned heparin) werden seit Ende der 1980er Jahre auch verschiedene fraktionierte Heparine oder Low Molecular Weight Heparine (=LMWH) eingesetzt. Fraktionierte Heparine ersetzen mittlerweile für viele Indikationen die UFH und werden durch chemische oder enzymatische Depolymerisation aus unfraktionierten Heparinen hergestellt, wobei Fragmente entstehen, die nur ca. 1/3 der Größe des Standard-Heparins haben. Hierdurch wird u.a. die Wirkung dieser LMWHs auf Thrombin abgeschwächt, während die Inaktivierung von Faktor Xa bevorzugt ist. Fraktionierte Heparine besitzen aufgrund ihrer vorteilhafteren Pharmakokinetik weitere Vorteile gegenüber den konventionellen unfraktionierten Heparinen. Ein Übersicht über diese Substanzklassen hinsichtlich ihrer klinischen Wirkung und Bedeutung ist in "Heparin and Low-Molecular-Weight Heparin (Mechanisms of Action, Pharmakokinetics, Dosing, Monitoring, Efficacy, and Safety)", Hirsh et al.; Chest 2001; 119:64S-94S zu finden.

Für Patienten, denen unfraktioniertes Heparin verabreicht wird, ist ein Monitoring aufgrund der individuellen Variabilität bei der Bioverfügbarkeit, der Proteinbindung und einer kurzen Halbwertszeit von 30-150 min vorgeschrieben, um eine eventuelle Überdosierung mit erhöhter Blutungsneigung oder Unterdosierung mit erhöhtem Thromboserisiko zu vermeiden. Die Überwachung der Therapie mit UFH erfolgt in der klinischen Routine am häufigsten mit der aktivierten partiellen Thromboplastinzeit (aPTT), aber auch mittels der Thrombin Clotting Time (TCT) oder der aktivierten Clotting Time (ACT). Diese Assays sind so genannte globale Assays, da sie unspezifisch die Thrombin-induzierte Bildung eines Fibrin-Clots reflektieren. Der aPTT-Test, welcher v.a. die Aktivität der Faktoren des intrinsischen Systems bestimmt, ist überwiegend sensitiv für die inhibierenden Effekte des Heparins auf Thrombin. Der aPTT-Test ist empfindlich für den Heparinbereich von 0,1-0,7 U/ml, wobei jedoch sowohl der Normbereich der aPTT als auch deren therapeutischer Bereich sehr stark vom Reagenz und dem verwendeten Analysegerät abhängig ist. Ein weiterer limitierender Faktor ist die Probenstabilität, die besonders nach zu langer Standzeit der Blutprobe zu oftmals verfälschten Ergebnissen führt. Weitere Nachteile solcher globaler Gerinnungsassays sind u.a. die oft komplexe Versuchsdurchführung, welche zur Erzielung reproduzierbarer Ergebnisse speziell geschultes Personal erfordert und der relative hohe Reagenzienverbrauch dieser Tests.

Aufgrund der besseren Pharmakokinetik ist ein Monitoring bei Gabe niedermolekularer Heparine beim Normalpatienten nicht zwingend. Eine Überprüfung der Therapie wird jedoch zu Beginn einer Behandlung empfohlen und ist insbesondere bei niereninsuffizienten Patienten aufgrund ihrer veränderten renalen Ausscheidung erforderlich sowie empfohlen bei Patienten mit extremem Körpergewicht, Neugeborenen, Kindern und Schwangeren oder bei mehrwöchiger Anwendung bzw. nach frischen Traumen oder Operationen.

In der klinischen Routine findet für das Monitoring von LMWH vor allem die aPTT Anwendung, obwohl dieser Test nur unzureichend sensitiv für dieses Antikoagulanz ist und zudem noch in sehr starkem Maße vom benutzten Nachweisreagenz abhängt.

Ein für ein Monitoring der Wirkung niedermolekularer Heparine geeigneter Nachweis-Test ist ein FXa-Test.

Bisherige FXa-Tests werden zumeist als chromogene Tests oder als Clotting Tests durchgeführt, wobei die chromogenen Tests Faktor-Xa-Aktivität und der Clotting Test Gerinnung messen. Beide Testprinzipien folgen demselben Testablauf:
1. FXa + [Heparin/Antithrombin III] → [FXa/Heparin/ATIII]-Komplex + restlicher FXa
2.
   a) restlicher FXa spaltet von FXa-spezifischem Substrat einen chromogenen Rest ab (chromogener Test)
   b) restlicher FXa spaltet Prothrombin zu Thrombin (Gerinnungstest über thrombininduzierte Fibrinvernetzung)

Bei Zugabe der Probe verbindet sich in definierter Menge im Testreagenz vorhandener Faktor Xa mit dem in der Probe enthaltenen Heparin und Antithrombin III und bildet mit diesen einen inaktivierten Komplex. Der restliche Faktor Xa spaltet entweder das chromogene Substrat oder bildet mit den übrigen in der Probe enthaltenen Gerinnungsfaktoren Thrombin, das Fibrinogen zu Fibrin spaltet (Clotbildung). Je nach Aktivität des Faktors Xa wird mehr oder weniger Substrat gespalten. Die Aktivität des Faktor Xa ist wiederum abhängig von der Menge des in der Probe enthaltenen Heparins. Während die chromogenen Tests spezifisch für die FXa-Aktivität in der Probe sind, messen Gerinnungstests nicht ausschließlich die FXa-Aktivität, sind jedoch oft dennoch sensitiver für LMWH als die aPTT.

Kommerziell sind mehrere chromogene Tests, aber nur wenige Gerinnungstests (Heptest der Fa. Sigma, ENOX-Test der Fa. Pharmanetics) verfügbar. Die verschiedenen Tests korrelieren untereinander und zur aPTT nur mäßig, da sie unterschiedliche Endpunkte haben. Die chromogenen Tests liefern Aktivitäten, keine Gerinnungszeiten. Die Korrelation zwischen Gerinnungszeit und Faktor Xa-Aktivität ist jedoch nur mäßig. Weiterhin erfordern diese chromogenen Tests eine Separation des Plasmas und können somit nicht direkt in Vollblutproben eingesetzt werden. Durch die aufwändigen Probenvorbereitungs- und Durchführungsschritte und die benötigten Vorrichtungen erfordern diese Bestimmungsmethoden einen hohen zeitlichen, personellen und apparativen Aufwand. Der Heptest liefert als Ergebnis zwar eine Gerinnungszeit, diese kann jedoch aufgrund der unterschiedlichen Heparinsensitivität des Patienten bei gleicher Heparinkonzentration sehr unterschiedlich ausfallen. Weiterhin ist die Erstellung einer Heparin-Eichkurve erforderlich, aus der dann das Testresultat abgelesen wird. Als trockenchemischer Test und somit auch für Point of Care-Geräte geeignet ist bislang nur ein einziger Faktor Xa-Test verfügbar (ENOX-Test der Fa. Pharmanetics), der mit einer Testkarte auf dem Rapid Point Analyzer der Fa. Bayer durchgeführt wird. Dieser Test wurde speziell für den Einsatz von Enoxaparin bei der Perkutanen Transluminalen Angioplastie entwickelt und unterscheidet lediglich zwischen Enoxaparin-Konzentrationen > 1 U/ml und < 1 U/ml und ist somit für das Routinemonitoring niedermolekularer Heparine nicht geeignet, da insbesondere andere fraktionierte Heparine und unfraktioniertes Heparin den Test stören.

### WO 03/050298 beschreibt das Prinzip dieses trockenchemischen FXa-Tests wie folgt:

Die zu untersuchende Probe, vorzugsweise citriertes Vollblut, wird mit einem trockenchemischen Reagenz versetzt, welches zumindest einen Faktor Xa-Aktivator, vorzugsweise Russels Viper Venom, sowie homogen verteilte magnetische Partikel enthält. Durch den im Reagenz enthaltenen Faktor Xa-Aktivator wird der in der Probe enthaltene Faktor X in Faktor Xa umgesetzt, welcher seinerseits über die Prothrombin-Thrombin-Umwandlung die Fibrinogenumsetzung zu Fibrin und damit die Ausbildung des Gerinnungs-Clots bewirkt. Der Nachweis dieses Clots erfolgt mittels optischer Methoden durch Beobachtung der Beweglichkeit der magnetischen Partikel im Reaktionsansatz, welche durch ein externes oszillierendes Magnetfeld hervorgerufen wird. Dieses Testprinzip beruht somit auf der Bildung eines Fibrin-Clots, welcher sich erst im Laufe der Nachweisreaktion in einer mehrstufigen, durch Faktor Xa ausgelösten Reaktionskaskade bildet, an welcher neben Faktor Xa weitere Enzyme und Cofaktoren beteiligt sind. So erfordert beispielsweise die Polymerisation und Quervernetzung des Fibrins die Anwesenheit von Calcium-Ionen und aktiviertem Faktor XIIIa, welcher wiederum durch eine Thrombin-abhängige Aktivierung aus inaktivem Faktor XIII gebildet wird. Die Bestimmung von Faktor Xa mittels der Bestimmung des Fibrin-Clots ist somit noch von weiteren essentiellen Faktoren und eventuellen Störeinflüssen abhängig. Neben dieser indirekten Bestimmungsmethode erfordert das in WO 03/050298 dargestellte Nachweisverfahren ein komplexes Nachweis- und Auswertesystem zur Faktor Xa-Bestimmung. So muss einerseits der Testträger, auf welchem die Gerinnungsreaktion stattfindet, spezielle Vorrichtungen aufweisen, welche eine gute und homogene Durchmischung der Reagenzien und Magnetpartikel mit der Probe gewährleisten und andererseits muß das Auswertesystem zur Bestimmung der Faktor Xa-Aktivität Vorrichtungen zur Erzeugung eines oszillierenden Magnetfelds, beispielsweise mittels eines beweglichen Permanentmagneten, und optische Systeme zur Beleuchtung und photometrischen Detektion der Bewegung der Magnetpartikel aufweisen.

WO 01/63271 beschreibt allgemein elektrochemische Sensoren auf trockenchemischer Basis zur Bestimmung der Blutgerinnung oder einzelner Gerinnungsfaktoren, welche auf einem inerten Träger mindestens 2 Elektroden aufweisen, sowie ein trockenes Reagens, welches ein Proteasesubstrat enthält, das aus einem Peptidrest besteht, der von einer Protease des Blutgerinnungssystems abgespalten werden kann, und über das Carboxylende amidisch an substituierte Amine, insbesondere an einen Phenylendiaminrest, gebunden ist. Diese substituierten Amine wirken nach der Protease-induzierten Abspaltung als Elektronenüberträger 2. Art und können zur elektrochemischen Bestimmung der Proteaseaktivität herangezogen werden. WO 01/63271 beschreibt neben den so genannten Globaltests wie aPTT, PT oder ACT, bei welchen die Gerinnungszeit über die Aktivität der Protease Thrombin ermittelt wird, auch Tests, mit welchen einzelne Gerinnungsfaktoren oder auch deren Inhibitoren bestimmt werden können. Hier lehrt WO 01/63271 den Einsatz von speziell auf den zu bestimmenden Gerinnungsfaktor zugeschnittenen Substraten, deren Peptidteil speziell an die zu bestimmende Protease angepasst ist, so dass es von dieser Protease spezifisch gespalten werden kann und das substituierte Amin als elektrochemisch detektierbares Teilchen spezifisch die Aktivität dieser Protease widerspiegelt. Übertragen auf einen Faktor Xa-Test würde dies bedeuten, ein Proteasesubstrat einzusetzen, welches aus einem Peptidrest besteht, der von Faktor Xa abgespalten werden kann, und über das Carboxylende amidisch an substituierte Amine, insbesondere an einen Phenylendiaminrest gebunden ist. Hierin ähnelt das Test-Prinzip den chromogenen Faktor Xa-Tests, bei welchen ebenfalls ein enzymatisches Abspaltungs-Produkt eines Faktor Xa-spezifischen Substrats zur Faktor Xa-Bestimmung eingesetzt wird.

### Aufgabe der Erfindung:

Aufgabe der vorliegenden Erfindung ist es, Verfahren und Vorrichtungen zur Bestimmung von Faktor Xa-Inhibitoren in Blutproben bereitzustellen, welche die Nachteile des Standes der Technik vermeiden.

Aufgabe der vorliegenden Erfindung ist es insbesondere, Verfahren zur Bestimmung von Faktor Xa-Inhibitoren in Blutproben bereitzustellen, welche einfach und mit geringem zeitlichen, apparativen oder personellem Aufwand auch von nicht speziell geschulten Personen durchgeführt werden können und in kurzer Zeit zu verlässlichen Ergebnissen führen.

Aufgabe der vorliegenden Erfindung ist es insbesondere, einfach durchzuführende Verfahren zur Bestimmung von Faktor Xa-Inhibitoren in Blutproben bereitzustellen, welche mit einer möglichst kleinen Zahl von Verfahrensschritten und benötigten Reagenzien und/oder Apparaten auskommt, so dass eine rasche dezentrale Analytik, beispielsweise direkt auf Intensiv- oder Krankenstationen, möglich wird.

Aufgabe der vorliegenden Erfindung ist es insbesondere, Verfahren und Vorrichtungen zur Bestimmung von Faktor Xa-Inhibitoren in Blutproben bereitzustellen, welche eine Bestimmung auch direkt im Vollblut ermöglichen und somit keine aufwändigen Probenvorbereitungsschritte benötigen.

Aufgabe der vorliegenden Erfindung ist es insbesondere, Verfahren und Vorrichtungen zur Bestimmung von Faktor Xa-Inhibitoren in Blutproben bereitzustellen, welchen den Anforderungen an Lagerbarkeit und Stabilität der Reagenzien genügen und eine möglichst genaue und spezifische Bestimmung ermöglichen.

Aufgabe der vorliegenden Erfindung ist es insbesondere, Verfahren und Vorrichtungen zur Bestimmung von Heparinen, insbesondere fraktionierten Heparinen oder Low Molecular Weight Heparinen, sowie direkten Faktor Xa-Inhibitoren in Blutproben bereitzustellen.

### Erfindungsgemäße Lösung:

Die vorliegende Erfindung stellt zur Lösung dieser Aufgaben Verfahren, elektrochemische Testelemente und Testelement-Analysesysteme zur Bestimmung von Faktor Xa-Inhibitoren in Blutproben bereit, wie sie in den unabhängigen Patentansprüchen genannt sind. Bevorzugte Ausführungsformen sind in den abhängigen Ansprüchen angegeben.

Insbesondere beschreibt die vorliegende Erfindung zur Lösung dieser Aufgaben Verfahren zur Bestimmung von Faktor Xa-Inhibitoren in Blutproben, welche dadurch gekennzeichnet sind, dass in einem ersten Schritt eine Probe des zu untersuchenden Blutes mit einem Nachweisreagenz, welches zumindest ein Thrombinsubstrat, welches aus einem Peptidrest besteht, der durch Thrombin abgespalten werden kann und der über das Carboxylende amidisch an eine elektrogene Substanz gebunden ist, enthält, und mit einer bekannten Menge von Faktor Xa in Kontakt gebracht wird, anschließend in einem zweiten Schritt die Menge oder Aktivität der elektrogenen Substanz, welche durch die Faktor Xa-vermittelte Thrombinaktivierung vom Thrombinsubstrat abgespalten wird, und/oder deren zeitlicher Verlauf als Messsignal mit elektrochemischen Methoden bestimmt wird, und schließlich in einem dritten Schritt anhand dieses Messsignals die Menge des Faktor Xa-Inhibitors in der Probe des zu untersuchenden Blutes oder eine damit korrelierende Messgröße, insbesondere eine damit korrelierende Gerinnungszeit, ermittelt wird.

Das erfindungsgemäße Verfahren wird anhand eines exemplarischen Beispiels näher erläutert:

Das Testprinzip beruht auf der Bestimmung der Enzymaktivität des durch die Faktor Xa-induzierte Gerinnungsreaktion gebildeten Thrombins mittels einer elektrochemischen Messung, wobei zumindest ein Spaltprodukt elektrochemisch an den Elektroden bestimmt wird. Als Thrombin-spezifisches Substrat kann insbesondere ein Tripeptid-Substrat wie beispielsweise reduziertes Chromozym TH (Tosyl-glycyl-prolyl-arginine-4-nitranilide acetate) eingesetzt werden, welches über das Carboxylende des Tripeptidteils amidisch eine elektrogene Substanz gebunden hat. Solche elektrogenen Substanzen sind beispielsweise in WO 01/63271 beschrieben und können insbesondere substituierte Aniline, insbesondere Nitroanilinderivate oder Phenylendiamine, sein. Die Nachweisreaktion läuft folgendermaßen ab:

Das Enzym Thrombin spaltet vom Substrat eine elektrogene Substanz, im vorliegenden Beispiel Phenylendiamin, ab, welches an den Elektroden zu Phenylendiimin oxidiert wird. In einer bevorzugten Ausführungsform findet an den Elektroden gleichzeitig mit der Oxidation dieses Spaltproduktes eine Reduktion einer Hilfssubstanz, beispielsweise von Resazurin zu Resorufin, statt, wodurch eine Stromstärke gemessen werden kann, welche mit der Spaltproduktmenge korreliert. So wird Resazurin in der vorliegenden elektrochemischen Reaktion als Hilfssubstanz an der Kathode reduziert, während an der Anode Phenylendiamin oxidiert wird. Für die elektrochemische Messung der Thrombinaktivität wird vorzugsweise ein Zwei-Elektroden-System eingesetzt, wobei das Potential an der Arbeitselektrode einerseits so hoch gewählt wird, dass das von Thrombin freigesetzte Phenylendiamin oxidiert wird und andererseits so niedrig gewählt wird, dass keine Restgruppe des Tripeptids reduziert wird. Die Potentialdifferenz zwischen der Kathode (Arbeitselektrode) und der Anode (Gegenelektrode) wird durch einen Potentiostaten geregelt. Als geeignete Potentialdifferenz kann im vorliegenden Beispiel beispielsweise eine Spannungsdifferenz von ca. 550 mV gewählt werden, um nur die gewünschten Reaktionen an den Elektroden zu ermöglichen.

In Bezug auf das erfindungsgemäße Verfahren wird dieses elektrochemische Testprinzip in einem Faktor Xa-Inhibitor-Test wie folgt angewendet:

Im Reagenz befindet sich neben den Nachweisreagenz eine bekannte Menge an Faktor Xa. Die zu untersuchende Blutprobe enthält den zu bestimmenden FXa-Inhibitor, welcher vorzugsweise ein Heparin ist. Dieses bildet mit dem im Reagenz befindlichen und in bekannter Menge im Überschuß vorliegenden Faktor Xa und Antithrombin III, welches in der Blutprobe vorliegt, einen stöchiometrischen Komplex. Hierbei bleibt eine FXa-Restmenge übrig, deren Konzentration von der Konzentration des Faktor Xa-Inhibitors in der Probe abhängig ist. Diese kann nun mit dem in der Blutprobe vorliegenden Prothrombin (und dem auch dort vorliegenden Faktor Va) zum Prothrombinkomplex und anschließend zum Thrombin reagieren, welches wie oben beschrieben über die thrombinbedingte enzymatische Abspaltung einer elektrogenen Substanz und deren elektrochemischen Nachweis bestimmt werden kann.

Je mehr Faktor Xa-Inhibitor in der Probe vorliegt, desto weniger Rest-FXa bleibt nach der Komplexbildung mit ATIII übrig und desto weniger Thrombin wird gebildet. Das vorliegende Messprinzip detektiert keine Gerinnungszeit im strikten Sinne, d.h. es handelt sich nicht um einen Clotting Test mit der Bildung von Fibrin, sondern um eine Bestimmung des zeitlichen Verlaufs der Thrombinbildung mit elektrochemischen Methoden.

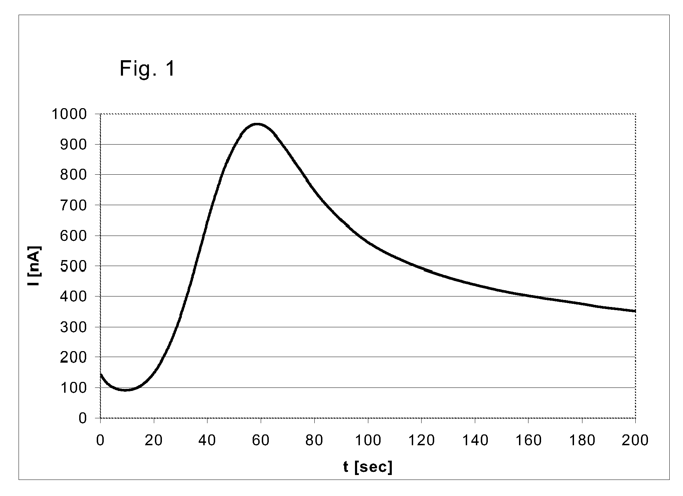

Beispiel 2 und Figur 1 zeigen exemplarisch das Ergebnis einer solchen elektrochemischen Thrombinbestimmung entsprechend den erfindungsgemäßen Verfahren.

Die erfindungsgemäße Verwendung von aktiviertem Faktor Xa mit einem Thrombin-Nachweisreagenz besitzt im Vergleich zu den chromogenen Faktor Xa-Tests und der in WO 01/63271 beschriebenen Verfahren eine höhere Aussagekraft über den physiologischen Zustand des Gerinnungssystems, da hier nicht ein künstliches Faktor Xa-spezifisches Substrat gespalten und darüber die Faktor Xa-Aktivität gemessen wird, sondern wie in der physiologischen Gerinnung zuerst Thrombin gebildet wird. Diese Bildung von Thrombin kann erfindungsgemäß mittels eines Thrombin-spezifischen Substrats elektrochemisch bestimmt und mit einem geeigneten Algorithmus beispielsweise in Gerinnungszeiten umgerechnet werden. Gegenüber Faktor Xa-Inhibitortests, welche wie der Heptest oder der ENOX-Test auf der Bestimmung der FXa-induzierten Bildung eines Fibrin-Clots beruhen, hat die Erfindung den Vorteil, dass die Bestimmung mittels elektrochemischer Methoden wesentlich einfacher und mit weniger personellem und apparativem Aufwand durchgeführt werden kann. Dadurch, dass die Faktor Xa-Aktivität mit den erfindungsgemäßen Verfahren durch die Aktivität des ersten in der physiologischen Gerinnungskaskade natürlicherweise vorkommenden nachgeschalteten Gerinnungsfaktors (Thrombin) bestimmt wird, wird einerseits die physiologische Aktivität des Faktor Xa-Inhibitors näher am natürlichen System bestimmt als mit chromogenen Tests und den in WO 01/63271 beschriebenen Verfahren, welche mit unphysiologischen Faktor Xa-Substraten arbeiten und andererseits wird der Nachweis der FXa-Aktivität an einer früheren Stelle der Gerinnungskaskade als bei den Tests, welche auf der Bestimmung der FXa-induzierten Bildung eines Fibrin-Clots beruhen, durchgeführt, so dass die zwischen der Thrombinbildung und der Fibrin-Clot-Bildung befindlichen, nachgeschalteten Gerinnungsfaktoren keinen Einfluss auf die Bestimmung der Faktor Xa-Aktivität haben. Hierdurch sind somit genauere und spezifischere Aussagen über die Faktor Xa-Aktivität im physiologischen System möglich als mit den bisher bekannten Tests.

Durch die erfindungsgemäße Lösung ist eine Faktor Xa-Inhibitor-Bestimmung insbesondere auch im Vollblut möglich, da anders als bei chromogenen Messmethoden eine Abtrennung störender, insbesondere farbiger, Blutbestandteile bei den genutzten elektrochemischen Methoden nicht notwendig ist. Durch eine auf die zum Nachweis genutzten elektrogenen Substanzen abgestimmte Ansteuerung der Elektroden, beispielsweise durch angepasste Wahl des Elektrodenpotentials, können die zum Nachweis genutzten elektrogenen Substanzen weitgehend unbeeinflusst von anderen in der Probe oder den Reagenzien vorkommenden und potentiell störenden Substanzen nachgewiesen werden.

Zur Bestimmung eines Faktor Xa-Inhibitors müssen erfindungsgemäß der zu untersuchenden Probe zumindest zwei Substanzen zugefügt werden oder in ihr vorhanden sein: eine bekannte Menge von Faktor Xa und als Nachweisreagenz ein peptidisches Thrombinsubstrat, aus welchem enzymatisch eine elektrogene Substanz abgespalten werden kann.

Viele der bekannten und in Thrombin-Tests eingesetzten Thrombinsubstrate, darunter auch das verbreitet eingesetzte Chromozym TH, weisen jedoch keine absolute und ausschließliche Thrombinspezifität auf, sondern können auch von anderen Enzymen gespalten werden.

Der Gerinnungsfaktor Xa ist eine Serinprotease, die natürlicherweise die Aminosäuresequenz -Ile-Glu-Gly-Arg- des Prothrombins erkennt und durch Spaltung an dieser Sequenz das natürliche Substrat Prothrombin zu Thrombin aktiviert. Neben diesem physiologischen Substrat hat Faktor Xa auch eine Fähigkeit, andere Peptide mit dieser oder auch anderen Spaltsequenzen enzymatisch zu spalten. So können auch peptidische Thrombinsubstrate, insbesondere das verbreitet eingesetzte Chromozym TH, von Faktor Xa, gespalten werden. Durch diese geringe Substratspezifität des Faktors Xa können in den erfindungsgemäßen Verfahren insbesondere dann Probleme auftreten, wenn ein solches auch von Faktor Xa spaltbares Thrombinsubstrat als Nachweisreagenz verwendet wird und dieses vor Zugabe der Probe bereits mit Faktor Xa in Kontakt kommt und von diesem umgesetzt werden kann. Hierdurch kann bereits ein Teil des Thrombinsubstrats umgesetzt werden, so dass dieser nicht mehr für die Thrombin-vermittelte Nachweisreaktion zur Verfügung steht und somit das Messresultat beeinflusst wird. Solche unerwünschten Nebenreaktionen können insbesondere dann auftreten, wenn Faktor Xa und das Thrombinsubstrat über einer längeren Zeitraum in Kontakt sind, wie dies beispielsweise der Fall wäre, wenn man diese beiden Substanzen gleichzeitig in ein flüssiges Nachweis-Reagenz bringen würde.

Bezogen auf das zuvor genannte exemplarische Messsystem würde dies bedeuten, dass im Falle, dass Faktor Xa und das als Thrombinsubstrat eingesetzte reduzierte Chromozym TH vor Beginn der Nachweisreaktion bereits über einen längeren Zeitraum miteinander reagieren konnten, bereits ein Teil des Thrombinsubstrats gespalten wäre und somit bereits eine weitgehend undefinierte Menge der abgespaltenen elektrogenen Substanz im Reagenz vorliegen würde. Solche Fälle können insbesondere dann vorliegen, wenn die beiden Stoffe gemeinsam in einem Flüssigreagenz vorliegen oder im Falle trockenchemischer Tests während derer Herstellung in flüssiger Form in Kontakt kamen. So kann eine gemeinsame Standzeit von 2h bereits ausreichen, um ca. 25% des vorhandenen reduzierten Chromozyms TH alleine durch die Faktor Xa-Wirkung zu spalten. Die Folge eines solchen Faktor Xa-bedingten vorzeitigen Substratabbaus wäre bei den elektrochemischen erfindungsgemäßen Verfahren insbesondere, dass das Messsignal bereits vor Beginn der Thrombin-bedingten Spaltung des reduzierten Chromozyms TH einen Versatz oder Offset zu höheren Stromstärken sowie auch hohe Stromstärken am Minimum aufweist, da in der Probe bereits durch die enzymatische Wirkung des Faktors Xa freigesetztes Phenylendiamin als elektrogene Substanz vorhanden ist.

Zur Vermeidung der Problematik des vorzeitigen Faktor Xa-bedingten Substratabbaus werden im Folgenden verschiedene Ausführungsformen als besonders bevorzugt dargestellt, mit welchen diese Problematik zumindest teilweise umgangen werden kann.

In einer bevorzugten Ausführungsform wird die Problematik des vorzeitigen Faktor Xa-bedingten Substratabbaus dadurch zumindest teilweise umgangen, dass eine bekannte Menge von Faktor Xa-Reagenz erst vor Beginn der Nachweisreaktion der Probe zugesetzt wird und so mit dieser in Kontakt gebracht wird.

In einer besonders bevorzugten Ausführungsform erfolgt dies dadurch, dass der Zusatz von Faktor Xa-Reagenz zur Probe räumlich oder zeitlich getrennt vom Zusatz des Nachweisreagenz zur Probe erfolgt.

In einer weiteren bevorzugten Ausführungsform erfolgt dies insbesondere dadurch, dass der Zusatz von Faktor Xa-Reagenz zur Probe räumlich oder zeitlich vor dem Zusatz des Nachweisreagenz zur Probe erfolgt.

Je kürzer Faktor Xa vor Beginn der Faktor Xa-Inhibitor-Messung mit dem Nachweisreagenz in Kontakt kommt, um so weniger kann Faktor Xa dieses unerwünschterweise umsetzen. Ein bevorzugter Ansatz ist es, Faktor Xa und das Nachweisreagenz möglichst nicht oder möglichst kurz vor Beginn der Faktor Xa-Inhibitor-Bestimmung miteinander in Kontakt zu bringen. Hierzu ist es erforderlich, dass das Faktor Xa-Reagenz und das Nachweisreagenz möglichst getrennt voneinander vorliegen und erst kurz vor Beginn der Faktor Xa-Inhibitor-Bestimmung miteinander in einer Weise in Kontakt kommen, dass sie miteinander reagieren können.

Eine Möglichkeit, dies zu erreichen, ist insbesondere bei nasschemischen Reaktionen der zeitlich versetzte Zusatz des Faktor Xa-Reagenz und des Nachweisreagenz zur Probe. Dies kann dadurch erfolgen, dass zunächst das Faktor Xa-Reagenz und anschließend das Nachweisreagenz zur Probe gegeben wird oder zunächst das Nachweisreagenz und anschließend das Faktor Xa-Reagenz zur Probe gegeben wird. Auch ist es möglich, dass das Nachweisreagenz und das davon zuvor getrennt gelagerte Faktor Xa-Reagenz gleichzeitig zur Probe gegeben werden. In einer weiteren Ausführungsform ist es auch möglich, dass Nachweisreagenz und Faktor Xa-Reagenz kurz vor Beginn der Faktor Xa-Inhibitor-Bestimmung miteinander vermischt werden und diese Mischung möglichst rasch zur Probe gegeben wird. In einer besonders bevorzugten Ausführungsform kann zunächst das Faktor Xa-Reagenz und anschließend das Nachweisreagenz zur Probe gegeben werden.

Bei trockenchemischen Nachweisverfahren und -vorrichtungen kann die zeitliche versetzte Zugabe von Faktor Xa-Reagenz und Nachweisreagenz zur Probe durch eine zumindest teilweise räumliche Trennung der beiden Reagenzien auf dem Testelement erreicht werden.

Trockenchemische Reagenzien werden meist dadurch auf Testelementen aufgebracht, dass sie in Form einer Lösung strichförmig auf das Testelement aufgebracht werden und dort das Lösungsmittel entzogen wird, so dass sie dort in trockenchemischer Form vorliegen. Dies ist besonders vorteilhaft, da Reagenzien in trockenchemischer Form oft wesentlich höheren Anforderungen hinsichtlich der Haltbarkeit und Stabilität genügen als nasschemische Reagenzien. Der Transfer in die aktivere, aber auch instabilere gelöste Form erfolgt zu gegebenem Zeitpunkt durch das Wiederauflösen der trockenchemischen Reagenzien durch Flüssigkeitszufuhr, vorzugsweise durch direktes Auflösen in der zu untersuchenden Blutprobe.

Durch die Möglichkeit, verschiedene Reagenzien in verschiedenen Kompartimenten auf einem Testelement anzuordnen, kann der Kontakt dieser Reagenzien bis zum Beginn der Faktor Xa-Inhibitor-Bestimmung weitgehend vermieden werden. Damit können auch unerwünschte Nebenreaktionen dieser Reagenzien weitgehend unterbunden werden. Vorzugsweise werden die in den verschiedenen Kompartimenten befindlichen Reagenzien erst durch die Probenflüssigkeit miteinander in Verbindung gebracht. In nasschemischen Verfahren oder Testelementen kann dies beispielsweise dadurch geschehen, dass die Reagenzlösungen in verschiedenen Flüssigkeitsräumen vorliegen und erst zu Beginn der Faktor Xa-Inhibitor-Bestimmung mit der Probe in Kontakt kommen, beispielsweise indem sie aus diesen Räumen der Probe mittels einer Pumpe hinzugefügt werden oder dass die unterschiedlichen Flüssigkeitsräume nacheinander von der Probe durchflossen werden.

Bei trockenchemischen Testelementen kann eine räumliche Trennung der Reagenzien vorzugsweise dadurch erzielt werden, dass Faktor Xa-Reagenz und Nachweisreagenz in räumlich voneinander getrennten Kompartimenten, vorzugsweise Reagenzstrichen, vorliegen, welche zur Bestimmung des Faktor Xa-Inhibitors von der flüssigen Probe aufgelöst werden und somit die darin enthaltenen Reagenzien in einen reaktiven Zustand versetzt werden. Die Trennung der Kompartimente muss hierbei nicht vollständig sein. So ist es beispielsweise möglich, Faktor Xa-Reagenz und Nachweisreagenz nebeneinander anzuordnen oder die Reagenzien in Form übereinander liegender Reagenzstriche anzuordnen, wobei die Überlagerung ganz oder auch nur teilweise sein kann. Hierzu sind Aufbringungsverfahren vorteilhaft, bei welchen zunächst ein erster Reagenzstrich aufgebracht und auf den Testelement getrocknet wird und in einem zweiten Schritt ein zweiter Reagenzstrich auf diesem aufgebracht und getrocknet wird, ohne dass durch das Aufbringen des zweiten Reagenzstriches der erste Reagenzstrich an- oder aufgelöst wird. Hierzu sind dem Fachmann verschiedene Methoden, beispielsweise die Verwendung bestimmter Zusatzstoffe wie Filmbildner oder Quellmittel, bekannt.

In einer bevorzugten Ausführungsform wird die räumliche Trennung der Reagenzien dadurch erzielt, dass die Reagenzstriche in Flussrichtung der Probe hintereinander angeordnet sind und somit deren Reagenzien durch die Probe nacheinander aktiviert werden, was einen zeitlich versetzten Zusatz zur Probe entspricht. Hierbei können die Reagenzstriche getrennt voneinander vorliegen oder auch in Kontakt zueinander stehen, beispielsweise nebeneinander liegen oder auch ganz oder teilweise versetzt übereinander aufgebracht sein.

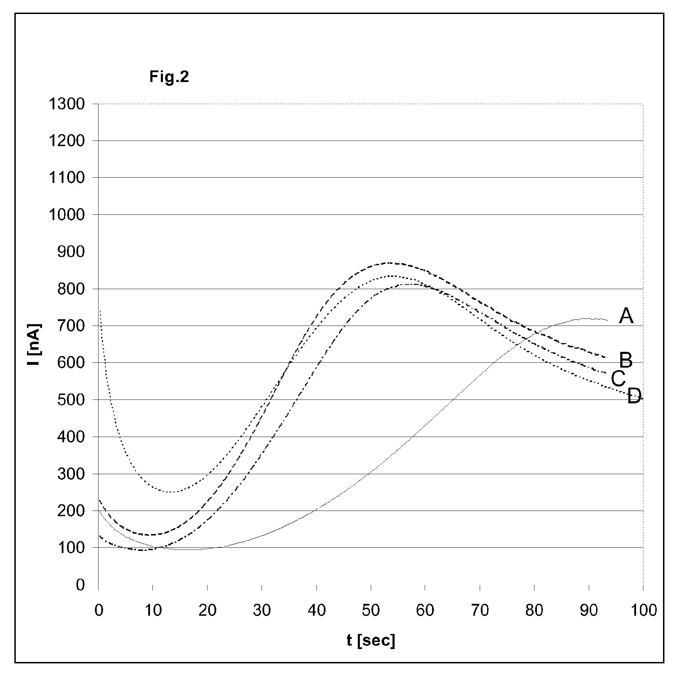

In einer besonders bevorzugten Ausführungsform ist der Reagenzstrich des Faktor Xa-Reagenzes zumindest teilweise in Flussrichtung der Probe vor dem Reagenzstrich des Nachweisreagenzes angebracht, bevorzugt in Form teilweise überlappender oder übereinander liegender Reagenzstriche, wobei in letzterem Fall der Reagenzstrich des Faktor Xa-Reagenzes über dem Reagenzstrich des Nachweisreagenzes aufgebracht ist. Durch diese erfindungsgemäße Anordnung zweier Reagenzstriche ist eine weitgehende Vermeidung unerwünschter Nebenreaktionen möglich, was sich, wie in Beispiel 3 und Figur 2 ersichtlich, in einem geringeren Eingangsstrom und geringeren Stromstärken im Minimum gegenüber einem Einstrichauftrag mit gemeinsam vorliegenden Faktor Xa- und Nachweisreagenz äußert. Durch die bevorzugte Anordnung des Reagenzstriches des Faktor Xa-Reagenzes über dem Reagenzstrich des Nachweisreagenzes können wesentlich kürzere von den Stromverläufen abgeleitete Zeitparameter als bei der umgekehrten Anordnung ermittelt werden, was für diagnostische Verfahren vorteilhaft ist.

Alternativ zu solchen Zwei-Strich-Aufträgen können auch Mischkammern verwendet werden, in welchen Faktor Xa-Reagenz und Nachweisreagenz erst kurz vor oder gemeinsam mit dem Aufbringen der Probe miteinander vermischt werden.

In einer weiteren bevorzugten Ausführungsform wird die Problematik des vorzeitigen Faktor Xa-bedingten Substratabbaus dadurch zumindest teilweise umgangen, dass eine bekannte Menge von Faktor Xa im Reaktionsansatz durch Zugabe einer bekannten Menge von Faktor X-Reagenz und eines Aktivator-Reagenz, welche die Umwandlung des proteolytisch inaktiven Faktors X in den proteolytisch aktiven Faktor Xa hervorruft, zur Probe erreicht wird.

Überraschenderweise hat sich durch dieses erfindungsgemäße Verfahren gezeigt, dass durch diese Reagenzienkombination ein besonders einfacher und stabiler Testelementaufbau möglich ist. Durch die Tatsache, dass im Reagenz vor dem Zusatz bzw. der Aktivierung eines Aktivator-Reagenzes kein aktivierter Faktor Xa, sondern inaktiver Faktor X vorliegt, ist es möglich, das Nachweisreagenz bereits vor Beginn der Bestimmungsreaktion mit Faktor X in Kontakt zu bringen, ohne dass es zu einem vorzeitigen Faktor Xa-bedingten Substratabbau und damit zu Beeinflussungen der Bestimmung kommt.

In besonders bevorzugten Ausführungsformen wird als Aktivator-Reagenz ein Aktivator oder ein beteiligter Komplex des extrinsischen Wegs der plasmatischen Gerinnung eingesetzt. Insbesondere können als Aktivator-Reagenzien natürlich vorkommende Aktivatoren oder Gerinnungsfaktoren wie Tissue Factor und/oder Faktor VIIa verwendet werden, wobei neben diesen natürlicherweise vorkommenden Aktivatoren oder Gerinnungsfaktoren auch andere, beispielsweise synthetisch hergestellte Aktivatoren eingesetzt werden können.

In weiteren bevorzugten Ausführungsformen wird als Aktivator-Reagenz ein Aktivator oder ein beteiligter Komplex des intrinsischen Wegs der plasmatischen Gerinnung eingesetzt. Insbesondere können als Aktivator-Reagenzien natürlich vorkommende Aktivatoren oder Gerinnungsfaktoren wie Faktor XIIa oder Substanzen, welche eine Umwandlung von Faktor X in Faktor Xa bewirken, beispielsweise Russels Viper Venom (RVV-X), eingesetzt werden, wobei neben diesen natürlicherweise vorkommenden Aktivatoren auch andere, beispielsweise synthetisch hergestellte Aktivatoren eingesetzt werden können.

Das Aktivator-Reagenz kann hierbei als externes Reagenz der Probe oder den anderen Reagenzien vor oder im Verlauf der Messung zugesetzt werden oder es kann bereits in der Blutprobe enthalten sein. Analoges gilt auch für weitere zum Ablauf der Faktor Xa-Aktivierung oder der Nachweisreaktion nötige Substanzen. So können insbesondere weitere benötigte Gerinnungsfaktoren wie Faktor V oder Antithrombin durch die zu untersuchende Blutprobe bereitgestellt werden.

In einer besonders bevorzugten Ausführungsform dieser Variante erfolgt der Zusatz von Faktor X-Reagenz zur Probe räumlich oder zeitlich getrennt vom Zusatz des Aktivator-Reagenz zur Probe.

Durch die Trennung von Faktor X-Reagenz und Aktivator-Reagenz wird eine unerwünschte vorzeitige Umsetzung des Faktors X zu Faktor Xa vermieden. Dies ist die Grundlage einer möglichst genau bekannten Faktor Xa-Menge zu Beginn der Messung, welche wiederum maßgeblich die Bestimmung des Faktor Xa-Inhibitors beeinflusst. Der Zusatz von Faktor X-Reagenz zur Probe erfolgt hierbei räumlich und/oder zeitlich getrennt vom Zusatz des Aktivator-Reagenz zur Probe.

Bevorzugte Verfahren und Vorrichtungen, welche einen räumlich oder zeitlich getrennten Zusatz von Aktivator-Reagenz und Faktor X-Reagenz zur Probe bewirken, können analog den zuvor beschrieben Verfahren und Vorrichtungen, welche einen räumlich oder zeitlich getrennten Zusatz von Nachweis-Reagenz und Faktor Xa-Reagenz zur Probe zu bewirken, ausgeführt werden, insbesondere in Form getrennter Reagenzstriche.

Zur erfindungsgemäßen Bestimmung von Faktor Xa-Inhibitoren muss neben dem Faktor X-Reagenz und dem Aktivator-Reagenz noch ein Nachweis-Reagenz vorliegen. Diese Reagenzien können sich vor Beginn der Bestimmung in zumindest teilweise räumlich getrennten Kompartimenten befinden. Da aber in dieser Ausführungsvariante kein Faktor Xa vor Beginn der Nachweisreaktion vorliegt, ist es in bevorzugten Ausführungsformen auch möglich, dass das Faktor X-Reagenz oder das Aktivator-Reagenz zusammen mit dem Nachweisreagenz vorliegt. Analoges gilt bei nachchemischen Verfahren für die Zusammenführung und Mischung der Reagenzien. So kann hier das Faktor X-Reagenz oder das Aktivator-Reagenz zusammen mit dem Nachweisreagenz in einer Reagenzlösung vorliegen. Die Bildung des Faktors Xa wird erst dann bewirkt, wenn die fehlende Reagenzkomponente (Faktor X-Reagenz oder Aktivator-Reagenz) dieser Reagenzienmischung zugesetzt wird.

Durch diese Reagenzienmischungen sind Bestimmungsverfahren und- vorrichtungen möglich, welche mit weniger Verfahrenschritten oder Reagenzienkompartimenten auskommen und somit leichter und kostengünstiger herstellbar sowie einfacher handhabbar sind.

In einer besonders bevorzugten Ausführungsform ist das Faktor X-Reagenz ein Bestandteil des Nachweisreagenz, wobei der Zusatz dieser Nachweisreagenz/Faktor X-Reagenz-Kombination zur Probe räumlich oder zeitlich getrennt vom Zusatz des Aktivator-Reagenz zur Probe erfolgt.

Bevorzugte Verfahren und Vorrichtungen, welche einen räumlich oder zeitlich getrennten Zusatz von Aktivator-Reagenz und Nachweisreagenz/Faktor X-Reagenz-Kombination zur Probe bewirken, können analog den zuvor beschriebenen Verfahren und Vorrichtungen, welche einen räumlich oder zeitlich getrennten Zusatz von Nachweis-Reagenz und Faktor Xa-Reagenz zur Probe zu bewirken, ausgeführt werden, insbesondere in Form getrennter Reagenzstriche.

Insbesondere kann der Zusatz der Nachweisreagenz/Faktor X-Reagenz-Kombination nach dem Zusatz des Aktivator-Reagenz erfolgen. In einer bevorzugten Ausführungsform wird die räumliche Trennung der Reagenzien dadurch erzielt, dass Reagenzstriche in Flussrichtung der Probe hintereinander angeordnet sind und somit deren Reagenzien durch die Probe nacheinander aktiviert werden, was einen zeitlich versetzten Zusatz zur Probe entspricht. Hierbei können die Reagenzstriche getrennt voneinander vorliegen oder auch in Kontakt zueinander stehen, beispielsweise nebeneinander liegen oder auch ganz oder teilweise versetzt übereinander aufgebracht sein. In einer besonders bevorzugten Ausführungsform ist der Reagenzstrich des Aktivator-Reagenzes zumindest teilweise in Flussrichtung der Probe vor dem Reagenzstrich der Nachweisreagenz/Faktor X-Reagenz-Kombination angebracht, bevorzugt in Form teilweise überlappender oder übereinander liegender Reagenzstriche, wobei in letzterem Fall der Reagenzstrich des Aktivator-Reagenzes über dem Reagenzstrich der Nachweisreagenz/Faktor X-Reagenz-Kombination aufgebracht ist.

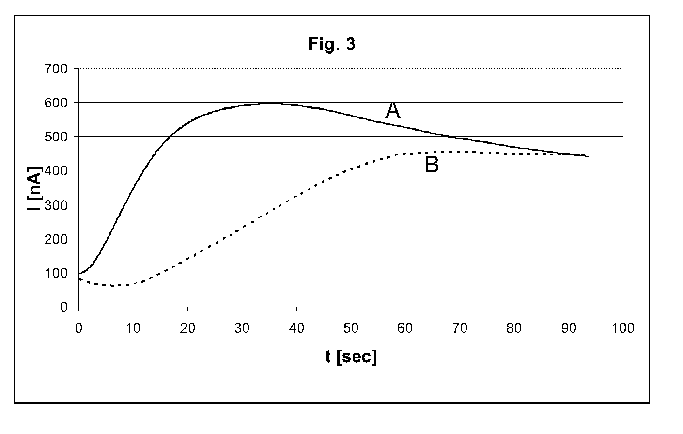

Beispiel 4 und Figur 3 zeigen Beispiele elektrochemischer Thrombinbestimmungen bei unterschiedlichen Anordnungen der Reagenzstriche des Aktivator-Reagenzes und der Nachweisreagenz/Faktor X-Reagenz-Kombination auf einem Testelement.

In einer weiteren bevorzugten Ausführungsform dieser Variante erfolgt der Zusatz von Faktor X-Reagenz zur Probe in einem gemeinsamen Schritt mit dem Zusatz des Aktivator-Reagenz zur Probe.

In nasschemischen Reaktionen kann dies beispielsweise dadurch erfolgen, dass die beiden Reagenzien gemeinsam aus ihrem jeweiligen Lagerungskompartimenten (Vorratsbehältern) zur Probe zugegeben werden, beispielsweise mittels Pumpen oder Pipetten. Es ist aber auch möglich, dass Faktor X-Reagenz und Aktivator-Reagenz erst zusammengegeben werden und diese Reagenzmischung, in welcher Faktor Xa entsteht, dann zur Probe gegeben wird.

In einer besonders bevorzugten Ausführungsform liegen Faktor X-Reagenz und Aktivator-Reagenz gemeinsam in einem trockenen Zustand vor und die Umwandlung von Faktor X in Faktor Xa wird erst durch den Kontakt mit der Probe bewirkt.

Durch die Verwendung von trockenchemischen Reagenzien ist es möglich, diese gemeinsam in einer inaktivierten Form zu lagern, ohne dass eine chemische Reaktion zwischen den Reagenzien stattfindet. Erst durch die Befeuchtung der trockenchemischen Reagenzien werden diese in einen Zustand gebracht, in welchem sie miteinander reagieren können. Diese "Aktivierung" der trockenchemischen Reagenzien wird in einer bevorzugten Ausführungsform durch die flüssige Blutprobe selbst bewirkt.

In einer besonders bevorzugten Ausführungsform liegen Faktor X-Reagenz, Aktivator-Reagenz und Nachweisreagenz gemeinsam als trockenchemische Reagenzien vor und die Umwandlung von Faktor X in Faktor Xa wird erst durch den Kontakt mit der Probe bewirkt.

Da es durch die Verwendung trockenchemischer Reagenzien möglich ist, diese gemeinsam in einer inaktivierten Form zu lagern, ohne dass eine chemische Reaktion zwischen den Reagenzien stattfindet, können mit diesen erfindungsgemäßen Verfahren und Vorrichtungen alle zur Bestimmung der FaktorXa-Inhibitoren notwendigen Reagenzien in einem gemeinsamen Kompartiment gelagert werden, ohne dass es zu einem Faktor Xa-bedingten vorzeitigen Thrombinsubstratabbau kommt. Erst durch die Zugabe der flüssigen Probe kommt es zu Umwandlung von Faktor X in Faktor Xa mit weitgehend definiertem Startpunkt und zeitlichem Verlauf, so dass eine bekannte Menge Faktor Xa in der Probe vorliegt, welche die Grundlage der Bestimmung von FaktorXa-Inhibitoren nach dem erfindungsgemäßen Prinzip bildet. Hierdurch lassen sich prinzipiell mit dem Fachmann bekannten Methoden alle zur Bestimmung von FaktorXa-Inhibitoren notwendigen Reagenzien in einem einzigen trockenchemischen Reagenzstrich oder -spot vereinen, was eine einfache und kostengünstige Produktion und eine einfache und weniger fehleranfällige Handhabung ermöglichen kann.

Die erfindungsgemäßen Verfahren und Vorrichtungen zur Bestimmung von Faktor Xa-Inhibitoren können insbesondere vorteilhaft zur Bestimmung von Heparinen, insbesondere fraktionierten oder niedermolekularen Heparinen, eingesetzt werden. Weitere Faktor Xa-Inhibitoren, welche mit den erfindungsgemäßen Verfahren und Vorrichtungen vorteilhaft bestimmt werden können, können insbesondere indirekte selektive oder direkte Faktor Xa-Inhibitoren sein.

Als Faktor Xa-Inhibitoren im Sinne der Erfindung können alle Substanzen angesehen werden, welche direkt oder indirekt die Aktivität des Faktors Xa beeinflussen, insbesondere erniedrigen und somit den Ablauf der Blutgerinnungskaskade beeinflussen, insbesondere verzögern. Direkte Faktor Xa-Inhibitoren wirken direkt auf den Faktor Xa und beeinflussen so dessen Aktivität, während indirekte Faktor Xa-Inhibitoren nicht direkt mit dem Faktor Xa wechselwirken, sondern dessen Menge und Bildung beeinflussen bzw. für ihre Aktivierung einen Cofaktor benötigen.. Dies können beispielsweise Antikoagulantien sein, welche auf Gerinnungsfaktoren wirken, welche in der Gerinnungskaskade vor Faktor Xa angesiedelt sind und zu dessen Bildung und/oder Regulierung beitragen. Beispiele für indirekte selektive Faktor Xa-Inhibitoren sind bestimmte Pentasaccharide wie Fondaparinux und Idraparinux, welche im Zusammenspiel mit Antithrombin III wirken und anders als die Heparine selektiv für Faktor Xa sind. Beispiele für direkte Faktor Xa-Inhibitoren ist Otamixaban oder Razaxaban.

Ein weiterer Aspekt der vorliegenden Erfindung betrifft Testelemente mit einem elektrochemischen Sensor auf trockenchemischer Basis zur Bestimmung von Faktor Xa-Inhibitoren, insbesondere unfraktionierten Heparinen, fraktionierten oder niedermolekularen Heparinen, indirekten selektiven Faktor Xa-Inhibitoren oder direkten Faktor Xa-Inhibitoren, der auf einem inerten Träger mindestens zwei Elektroden aufweist und ein Nachweisreagenz, welches zumindest ein Thrombinsubstrat, welches aus einem Peptidrest besteht, der durch Thrombin abgespalten werden kann und der über das Carboxylende amidisch an eine elektrogene Substanz gebunden ist, sowie zumindest eine bestimmte Menge von Faktor X-Reagenz oder Xa-Reagenz enthält.

Solche erfindungsgemäßen Testelemente eignen sich besonders, die zuvor beschriebenen erfindungsgemäßen Verfahren zur Bestimmung von Faktor Xa-Inhibitoren in Blutproben durchzuführen.

Erfindungsgemäße Testelemente sind insbesondere elektrochemische Testelemente auf trockenchemischer Basis. Solche Testelemente enthalten mindestens zwei Elektroden, von denen mindestens eine Elektrode eine so genannte Arbeitselektrode ist. Sie enthalten nicht notwendigerweise eine klassische Referenzelektrode, wie beispielsweise eine Ag/AgCl-Referenzelektrode, sondern lediglich mindestens eine Arbeits- und eine Gegenelektrode. Die Elektroden können aus allen gängigen Elektrodenmaterialien, beispielsweise Metallen, Edelmetallen, Legierungen oder Graphit aufgebaut sein, vorzugsweise aus Edelmetallen, wie Gold oder Palladium, oder Graphit. Die unterschiedlichen Elektroden des Testelements können aus gleichen oder unterschiedlichen Materialien bestehen. In einer besonders bevorzugten Ausführungsform enthält das Testelement eine Arbeitselektrode und eine Gegenelektrode, die beide aus Gold bestehen.

Konkrete Ausgestaltungen solcher elektrochemischer Testelemente, insbesondere hinsichtlich Wahl der Materialien, Anordnung der Elektroden und Reagenzien sowie Herstellung der Testelemente, sind im Stand der Technik, beispielsweise in US 5762770, US 6270637 oder WO 01/63271 beschrieben und dem Fachmann bekannt.

Die Reagenzien werden in bevorzugten Ausführungsformen in Form eines oder mehrerer Reagenzstriche auf die Elektroden aufgebracht. Diese können getrennt voneinander vorliegen oder sich teilweise oder komplett überdecken. Weiterhin ist es erfindungsgemäß möglich, die Reagenzien nicht direkt auf die Elektroden, sondern in der Nähe der Elektroden, beispielsweise neben den Elektroden auf einem flachen Substrat, aufzubringen und zu trocknen. In diesem Fall werden die Reagenzien mit der Probe während der Messung zu den Elektroden transportiert. Alternativ dazu ist es möglich, die Reagenzien in porösen Materialien wie zum Beispiel Vliesen, Papieren, Membranen und dergleichen unterzubringen, beispielsweise ablösbar zu imprägnieren. Diese Materialien müssen dabei von der Probe vor dem Kontakt mit den Elektroden durchströmt werden und dabei die Reagenzien in die Probe abgeben können. Es ist auch möglich, einzelne Bestandteile der Reagenzien auf unterschiedliche Kompartimente des Testelements aufzubringen, beispielsweise das Nachweisreagenz auf oder direkt neben die Elektrode, ein Faktor Xa-Reagenz oder ein Faktor X-Reagenz und ein Aktivator-Reagenz aber räumlich getrennt davon (z.B. weiter entfernt von der Elektrode), oder in unterschiedliche Schichten (z. B. Membranen oder Vliese) zu imprägnieren.

In einer bevorzugten Ausführungsform enthält das Testelement Faktor Xa-Reagenz, wobei das Faktor Xa-Reagenz auf dem Testelement zumindest teilweise räumlich getrennt vom Nachweisreagenz vorliegt, insbesondere zumindest teilweise in Flussrichtung der Probe vor dem Nachweisreagenz angeordnet ist.

In einer besonders bevorzugten Ausführungsform wird dies beispielsweise dadurch erreicht, dass Faktor Xa-Reagenz und Nachweisreagenz in Form zweier Reagenzstriche auf dem Testelement angeordnet sind. Diese können getrennt voneinander vorliegen oder in Kontakt miteinander sein. Besonders bevorzugt ist eine Anordnung, bei welcher der Reagenzstrich des Faktor Xa-Reagenz zumindest teilweise in Flussrichtung der Probe vor dem Reagenzstrich des Nachweisreagenzes angeordnet ist und mit diesem zumindest teilweise in Kontakt steht, insbesondere diesen zumindest teilweise überdeckt.

In einer weiteren Ausführungsform enthält das Testelement Faktor X-Reagenz und ein Aktivator-Reagenz, welches die Umwandlung von Faktor X in Faktor Xa hervorruft, wobei das Aktivator-Reagenz auf dem Testelement zumindest teilweise räumlich getrennt vom Faktor X-Reagenz vorliegt, insbesondere zumindest teilweise in Flussrichtung der Probe vor dem Faktor X-Reagenz angeordnet ist.

In einer bevorzugten Ausführungsform wird dies beispielsweise dadurch erreicht, dass Faktor X-Reagenz und Aktivator-Reagenz in Form separater Reagenzstriche auf dem Testelement angeordnet sind. Diese können getrennt voneinander vorliegen oder in Kontakt miteinander sein. Besonders bevorzugt ist eine Anordnung, bei welcher der Reagenzstrich des Aktivator-Reagenzes zumindest teilweise in Flussrichtung der Probe vor dem Reagenzstrich des Faktor X-Reagenzes angeordnet ist und mit diesem zumindest teilweise in Kontakt steht, insbesondere diesen zumindest teilweise überdeckt. Das Nachweisreagenz liegt bei dieser Ausführungsform in einem weiteren Kompartiment, insbesondere einem weiteren Reagenzstrich vor, welcher allerdings mit den Reagenzstrichen des Aktivator-Reagenzes und des Faktor X-Reagenzes zumindest teilweise in Kontakt stehen kann.

In einer besonders bevorzugten Ausführungsform liegt das Nachweisreagenz gemeinsam mit dem Aktivator-Reagenz oder dem Faktor X- Reagenz auf dem Testelement vor. Da weder Aktivator-Reagenz oder Faktor X- Reagenz für sich alleine aktivierten Faktor Xa besitzen oder bilden können, kann das Nachweisreagenz einem dieser Reagenzien zugemischt werden, ohne dass es zu einem vorzeitigten Faktor Xa-bedingten Abbau des Nachweisreagenz und damit zu unerwünschten Beeinflussungen der Faktor Xa-Inhibitor-Bestimmungen kommen kann. Durch die Zusetzung des Nachweisreagenzes zu einem der beiden anderen Reagenzien kann auf ein Reagenzkompartiment verzichtet werden, wodurch einfachere und kostengünstiger herzustellende Testelementaufbauten möglich sind. In einer besonders bevorzugten Ausführungsform kann dies beispielsweise durch eine Anordnung erreicht werden, bei welcher der Reagenzstrich des Aktivator-Reagenzes zumindest teilweise in Flussrichtung der Probe vor dem Reagenzstrich des Faktor X-Reagenzes angeordnet ist und mit diesem zumindest teilweise in Kontakt steht, insbesondere diesen zumindest teilweise überdeckt und das Nachweisreagenz gemeinsam mit dem Faktor X-Reagenz in einem Reagenzstrich vorliegt.

In einer weiteren Ausführungsform enthält das Testelement Faktor X-Reagenz und Aktivator-Reagenz in trockenchemischer Form, welches die Umwandlung von Faktor X in Faktor Xa hervorruft, wobei das Aktivator-Reagenz auf dem Testelement gemeinsam mit dem Faktor X-Reagenz, optional auch gemeinsam mit dem Nachweisreagenz, vorliegt und wobei die Umwandlung von Faktor X in Faktor Xa erst durch den Kontakt mit der Probe bewirkt wird. Dadurch, dass Faktor X-Reagenz und Aktivator-Reagenz in trockenchemischer Form auf dem Testelement vorliegen, liegen diese in einer Form vor, in welcher sie nicht miteinander reagieren können. Dadurch kann Faktor X-Reagenz und Aktivator-Reagenz gemeinsam in einem Kompartiment gelagert werden, ohne dass Faktor Xa gebildet wird. Erst durch den Zusatz von Flüssigkeit, insbesondere durch den Kontakt mit der Probenflüssigkeit, können die beiden Reagenzien miteinander reagieren und es kann zur Umwandlung von Faktor X in Faktor Xa kommen.

In einer bevorzugten Ausführungsform wird dies beispielsweise dadurch erreicht, dass Faktor X-Reagenz und Aktivator-Reagenz in Form eines gemeinsamen Reagenzstriches zusammen auf dem Testelement angeordnet sind. Das Nachweisreagenz kann bei dieser Ausführungsform in einem weiteren Kompartiment vorliegen, insbesondere einem weiteren Reagenzstrich, welcher allerdings mit dem Reagenzstrich des kombinierten Aktivator- /Faktor X-Reagenzes zumindest teilweise in Kontakt stehen kann.

In einer besonders bevorzugten Ausführungsform kann auch das Nachweisreagenz dem Faktor X-Reagenz und dem Aktivator-Reagenz zugesetzt sein, so dass alle zur Bestimmung von Faktor Xa-Inhibitoren benötigten Reagenzien in einem einzigen Kompartiment, insbesondere einem einzigen Reagenzstrich, auf dem Testelement vorliegen können. Wie zuvor ausgeführt, ist es durch die Verwendung trockenchemischer Reagenzien möglich, Faktor X-Reagenz und Aktivator-Reagenz gemeinsam in einem Kompartiment zusammenzubringen, ohne dass Faktor Xa gebildet wird. Hierdurch ist es nun möglich, auch das Nachweisreagenz zu dieser Reagenzienkombination zu geben, da ohne aktivierten Faktor Xa auch kein vorzeitiger Faktor Xa-bedingter Abbau des Nachweisreagenzes erfolgen kann. Insbesondere ist es durch diese erfindungsgemäße Lösung möglich, alle zur Bestimmung von Faktor Xa-Inhibitoren notwendigen Reagenzien in einem einzigen Kompartiment aufzubringen, wodurch einfachere und kostengünstiger herzustellende Testelementaufbauten möglich sind.

Ein weiterer Aspekt der vorliegenden Erfindung betrifft elektrochemische Testelement-Analysesysteme, welche zumindest ein Testelement gemäß den Patentansprüchen und ein Strommessgerät enthalten. Durch die Verwendung solcher Messgeräte können die beim Ablauf der Faktor Xa-Inhibitor-Bestimmung entstehenden elektrogenen Thrombinsubstrat-Spaltprodukte, insbesondere Phenylendiamin, mittels des an den Elektroden stattfindenden Stromflusses erfasst werden. In besonders bevorzugten Ausführungsformen wird der Verlauf des Stromflusses über die Zeit erfasst. Die elektrochemische Bestimmung erfolgt vorzugsweise quasi-potentiostatisch, bevorzugt mit einem 2-Elektrodensystem, bei dem die eine Elektrode gleichzeitig als Referenz- und Gegenelektrode, die andere Elektrode als Arbeitselektrode geschaltet ist. An dieses 2-Elektrodensystem wird eine konstante Spannung angelegt und der Strom über die Zeit gemessen. Dieses Verfahren wird auch als amperometrisches Messverfahren bezeichnet. Hierbei wird der zeitliche Stromverlauf erfasst und ermittelt, nach welcher Zeitspanne ab dem Start der Bestimmungsreaktion der gemessene Strom einen vorbestimmten Schwellenwert überschreitet. Diese Zeitspanne ist ein Maß für die Faktor Xa-bedingte Thrombinsubstratumsetzung im Laufe der Gerinnungsreaktion, welche wiederum von der Menge des in der Probe vorhandenen Faktor Xa-Inhibitors abhängt.

Neben dem beschriebenen amperometrischen Messverfahren können auch voltametrische Messverfahren eingesetzt werden. Hierbei wird keine konstante Spannung zwischen den Elektroden geregelt, sondern die Spannung wird linear von einem Startwert zu einem Endwert verändert und anschließend wieder zum Startwert zurück gefahren. Dieser Vorgang kann mehrfach über die gesamte Messdauer wiederholt werden. Beim voltametrischen Verfahren wird der Strom gegen die Spannung aufgetragen und man erhält dann entsprechend den Wiederholungen ineinander geschachtelte Strom-Spannungskurven (Zyklovoltamogramme). Bei geeignetem Spannungsbereich bilden sich Oxidationspeaks und Reduktionspeaks des Elektronenüberträgers in diesen Kurven ab. Die Höhe dieser Peaks ist direkt proportional der Konzentration des Elektronenüberträgers, sofern nicht weitere redoxaktive Substanzen im überdeckten Potentialbereich mit oxidiert oder reduziert werden und damit einen zusätzlichen Strombeitrag liefern. Bei der Messung einer Konzentrationsänderung kann eine solche Störung gegebenenfalls vernachlässigt werden. Trägt man nun die Stromwerte der Peak-Maxima oder die durch die Kurven eingeschlossenen Flächen (die dem Ladungsumsatz entsprechen) der einzelnen Strom-Spannungsschleifen über die Zeit auf, erhält man ebenfalls ein Bild der Konzentrationsänderung des Elektronenüberträgers über die Messdauer in einem durch die Zyklusdauer gegebenen Zeitraster. Diese Information kann dann - wie bei den amperometrischen Verfahren - zur Bestimmung des Faktor Xa-Inhibitors herangezogen werden. Solche Verfahren sind beispielsweise in WO 01/63271 beschrieben.

Die erfindungsgemäßen Verfahren und Vorrichtungen können insbesondere zur Bestimmung von Faktor Xa-Inhibitoren in Blutproben eingesetzt werden. Im Sinne der vorliegenden Anmeldung umfasst der Begriff Blutprobe nicht nur unbehandeltes Blut (Vollblut), sondern auch Blutderivate oder Blutprodukte, welche durch physikalische und/oder chemische und/oder biochemische Weiterbehandlung aus nativen Blutproben gewonnen werden können. Als Blutderivate können im Sinne der vorliegenden Anmeldung insbesondere Serum oder Plasma angesehen werden. Unter Bestimmung von Faktor Xa-Inhibitoren kann im Sinne der vorliegenden Anmeldung jede qualitative, quantitative oder semiquantitative Bestimmung von Faktor Xa-Inhibitoren gelten.

### Ausführungsbeispiele

Die Erfindung wird durch nachfolgende exemplarische Beispiele und Abbildungen näher erläutert.
Figur 1 zeigt exemplarisch das primäre Messergebnis einer elektrochemischen Faktor Xa-Inhibitor-Bestimmung entsprechend den erfindungsgemäßen Verfahren.
Figur 2 zeigt die Ergebnisse elektrochemischer Thrombinbestimmungen bei unterschiedlichen Anordnungen der Reagenzstriche eines Faktor Xa-Reagenzes und eines Nachweisreagenzes auf einem Testelement.
Figur 3 zeigt die Ergebnisse elektrochemischer Thrombinbestimmungen bei unterschiedlichen Anordnungen der Reagenzstriche eines Aktivator-Reagenzes und einer Nachweisreagenz/Faktor X-Reagenz-Kombination auf einem Testelement
Figur 4 zeigt die Ergebnisse elektrochemischer Faktor Xa-Inhibitor-Bestimmungen von Vollblutproben unterschiedlicher Faktor Xa-Inhibitor-Konzentration.

### Beispiel 1: Exemplarischer Aufbau eines erfindungsgemäßen Testelements

Ein möglicher Aufbau eines erfindungsgemäßen Testelements und dessen Fertigung wird im Folgenden exemplarisch beschrieben.

Das inerte Trägermaterial kann beispielsweise aus einer Polyesterfolie, beispielsweise einer Melinexfolie, bestehen. Auf diese können mit verschiedensten Verfahren Elektrodenstrukturen aufgebracht werden, beispielsweise durch Bedampfung der Trägerfolie mit Gold und anschließender Laserablation der gewünschten Elektrodenstrukturen, wobei dem Fachmann auch noch andere Verfahren wie beispielsweise Ätzverfahren oder Druckverfahren zur Herstellung dieser Elektrodenstrukturen geläufig sind. Optional können anschließend bestimmte Teile der Elektrodenstrukturen mit isolierenden Schichten versehen werden.

Auf das Trägermaterial mit seinen Elektrodenstrukturen werden nun die zur Bestimmung benötigten Reagenzien aufgebracht. Dies kann mit verschiedenen dem Fachmann bekannten Methoden geschehen, insbesondere mit Druck- oder Rakelverfahren, bei welchen die Reagenzien in gelöster Form in Form von Reagenzstrichen auf das Testelement aufgebracht werden und anschließend das Lösungsmittel zumindest teilweise entzogen wird, so dass die Reagenzien in trockenchemischer, d.h. inaktivierter Form, auf dem Testelement vorliegen. Solche Verfahren sind beispielsweise in WO 01/63271 beschrieben.

Üblicherweise werden bei solchen Beschichtungsverfahren die Reagenzien in einem Grundansatz gelöst, welcher Substanzen enthält, welche eine möglichst optimale Verarbeitung der Reagenzlösung ermöglichen.

Eine exemplarische Zusammensetzung eines solchen Grundansatzes ist beispielsweise (gelöst in Aqua bidest):

| Konzentration (in Klammern: erfindungsgemäß möglicher Bereich) | Substanz | Funktion der Substanz | Lieferant |
|---|---|---|---|
| 3,5 g/l (0-50 g/l) | Keltrol F | Filmbildner | Roche Diagnostics GmbH |
| 5 g/l (0-75 g/l) | Hydroxypropylzellulose | Verdicker | Hercules |
| 1 g/l (0-10 g/l) | Mega 8 | Detergenz | Roche Diagnostics GmbH |
| 20 g/l (0-200 g/l) | Mowiol | Filmbildner | Kuraray |
| | Grundpuffer | | |
| 120 mM (10-500mM) | HEPES | pH-Puffer | Sigma |
| 10 g/l (0,1-100 g/l) | RPLA 4 (Serumalbumin) | Schutzprotein | Roche Diagnostics GmbH |
| 30 g/l (0-200 g/l) | Glycin | Stabilisator, Lösungsvermittler | Roche Diagnostics GmbH |
| 75 g/l (0-300 g/l) | Sucrose | Stabilisator, Lösungsvermittler | Roche Diagnostics GmbH |

Zu diesem Grundansatz werden nun jeweils die spezifischen Reagenzkomponenten (z.B. Thrombinsubstrat, Faktor X, Faktor Xa, Aktivator-Reagenz; einzeln oder auch in Kombination) zugemischt und räumlich definiert, beispielsweise als Reagenzstriche oder -spots, auf das Testelement aufgebracht. Nach Entzug des Lösungsmittels liegen nun definierte und stabile Reagenzstriche oder -spots auf dem Testelement vor, in welchen die Reagenzien in stabiler Form über einen langen Zeitraum ohne Aktivitätsverlust gelagert werden können. In bevorzugten Ausführungsformen werden auf die Testträger weiterhin seitliche Abstandshalter und eine Deckelfolie aufgebracht, welche einen Kapillarkanal definieren, durch welcher ein weitgehend definiertes Volumen der Probe aufgenommen und zu den Reagenzkompartimenten und Elektroden transportiert werden kann.

### Beispiel 2: Exemplarische Durchführung einer elektrochemischen Faktor Xa-Inhibitor-Bestimmung entsprechend den erfindungsgemäßen Verfahren und Vorrichtungen

Auf ein erfindungsgemäßes Testelement, wie es vorzugsweise nach Beispiel 1 hergestellt werden kann, wird zur Durchführung der Faktor Xa-Inhibitor-Bestimmung eine ausreichende Menge, beispielsweise 1 - 1000 µl, bevorzugt ca. 10 µl, des zu untersuchenden Blutes in einer Weise aufgebracht, dass es mit den auf dem Testelement befindlichen Reagenzkompartimenten und Elektroden in Kontakt kommt. Dies kann bevorzugterweise durch das Vorhandensein eines Kapillarkanals auf dem Testelement und geeignete Positionierung der Reagenzkompartimente und Elektroden auf dem Testelement innerhalb dieses Kapillarkanals bewirkt werden. In bevorzugten Ausführungsformen können Probenflüssigkeit, Testelement und/oder sonstige zur Faktor Xa-Inhibitor-Bestimmung notwendigen Vorrichtungen auf eine bestimmte Temperatur, insbesondere eine Temperatur von ca. 37°C, temperiert sein. Das Testelement wird zur Erfassung der elektrochemischen Nachweisreaktionen an ein geeignetes Messgerät angeschlossen, beispielsweise ein Strommesssystem, welches insbesondere den zeitlichen Verlauf des Stromflusses erfassen und speichern kann.

Figur 1 zeigt exemplarisch den zeitlichen Verlauf einer elektrochemischen Faktor Xa-Inhibitor-Bestimmung entsprechend den erfindungsgemäßen Verfahren. Hierbei wurde ein Testelement entsprechend Beispiel 1 eingesetzt.

Die Kurve zeigt den zeitlichen Verlauf der gemessenen Stromstärke, bedingt durch die Oxidation des Phenylendiamins nach dessen Thrombin-induzierter Abspaltung aus dem Thrombin-Substrat. Auf der x-Achse ist die Zeit t in Sekunden nach der Auftragung der Blutprobe, auf der y-Achse die zum jeweiligen Zeitpunkt bestimmte Stromstärke I in Nanoampere aufgetragen. Die Kurve durchläuft zunächst ein Minimum, steigt dann mit zunehmender Oxidation des Phenylendiamins an, erreicht schließlich ein Maximum und fällt dann aufgrund der Substratverarmung kontinuierlich ab. Die Auswertung solcher zeitlichen Stromverläufe zur Bestimmung von zeitlichen Messgrößen, welche den Ablauf der Nachweisreaktion widerspiegeln, erfolgt beispielsweise mittels eines Schwellwertalgorithmus. Dieser Algorithmus addiert auf das errechnete Minimum einen Schwellwert, beispielsweise 60 nA, der für alle Messungen beibehalten wird. Als zeitliche Messgröße für die Thrombinumsetzung kann die Zeit nach Erreichen des Minimums bestimmt werden, bis zu welcher dieser Schwellwert erreicht wird. Da die so ermittelten zeitlichen Messgrößen für die Thrombinumsetzung von der Aktivität/Konzentration des aktivierten Faktors Xa und diese wiederum von der Menge bzw. Konzentration evtl. Faktor Xa-Inhibitoren abhängig sind, kann von den so ermittelten Zeitwerten auf das Vorhandensein, das Über- oder Unterschreiten einer bestimmten Konzentration bzw. die Menge oder die Konzentration eines Faktor Xa-Inhibitors geschlossen werden. Hierzu können beispielsweise Kurven mit Proben bekannter Faktor Xa-Inhibitor-Konzentration erstellt werden, welche diese Konzentrationen mit deren erfindungsgemäß ermittelten Zeitparametern in Korrelation setzen.

Weiterhin können aus diesen bestimmten zeitlichen Messgrößen weitere damit und mit der Menge eines evtl. vorhandenen Faktor Xa-Inhibitors korrelierende Messgrößen, insbesondere Gerinnungszeiten, unter Verwendung geeigneter Algorithmen ermittelt werden.

### Beispiel 3: Einfluss der Anordnungen der Reagenzstriche auf dem Testelement, welches Faktor Xa-Reagenz enthält

Figur 2 zeigt die Ergebnisse elektrochemischer Thrombinbestimmungen bei unterschiedlichen Anordnungen der Reagenzstriche eines Faktor Xa-Reagenzes und eines Nachweisreagenzes auf einem Testelement. Dargestellt ist jeweils der zeitliche Verlauf der gemessenen Stromstärke, wobei auf der x-Achse die Zeit t in Sekunden nach der Auftragung der Blutprobe und auf der y-Achse die zum jeweiligen Zeitpunkt bestimmte Stromstärke I in Nanoampere aufgetragen ist. Im vorliegenden Beispiel enthielt der Reagenzstrich des Faktor Xa-Reagenzes jeweils 2U/mI Faktor Xa (factor Xa der Firma american diagnostica) und zusätzlich 0,04 % Phospholipide (PHL, Sojabohnen Lecithin der Firma Sigma), der Reagenzstrich des Nachweisreagenzes 0,8 ml reduziertes Chromozyms TH (Pefachrom FXa der Firma Pentapharm Ltd.). Die Reagenzstriche wurden in folgenden Varianten aufgetragen:
Kurve A: Zuerst wurde der Reagenzstrich des Faktor Xa-Reagenzes aufgetragen, über diesen dann der Reagenzstrich des Nachweisreagenzes.
Kurve B: Zuerst wurde der Reagenzstrich des Nachweisreagenzes aufgetragen, über diesen dann der Reagenzstrich des Faktor Xa-Reagenzes.
Kurve C: Zuerst wurde der Reagenzstrich des Nachweisreagenzes aufgetragen, dann der Reagenzstrich des Faktor Xa-Reagenzes, wobei die Auftragung des Reagenzstriches des Faktor Xa-Reagenzes versetzt erfolgte, so dass dieser zumindest teilweise in Flussrichtung der Probe vor dem Reagenzstrich des Nachweisreagenzes liegt und diesen zumindest teilweise überdeckt.
Kurve D: Als Referenz wurde ein Einstrichauftrag gewählt, wobei die Reagenzien des Reagenzstrichs des Faktor Xa-Reagenzes und des Reagenzstrichs des Nachweisreagenzes vor der Auftragung vermischt wurden und in Form eines einzigen Striches aufgetragen wurden.

Figur 2 zeigt bei den 2-Strichaufträgen (Kurven A-C) keinen Versatz des Minimums zu höheren Stromwerten, wie dies beim 1-Strichauftrag (Kurve D) auftritt. Es zeigt sich weiterhin, dass der Eingangsstrom des 1-Strichauftrags mit 750 nA gegenüber 120-220 nA beim 2-Strichauftrag deutlich erhöht war. Dies deutet darauf hin, dass der vorzeitige Substratabbau durch die erfindungsgemäße zumindest teilweise räumliche Trennung der Reagenzien in zwei Kompartimente, insbesondere Reagenzstriche, größtenteils unterbunden werden kann.

Bei der Betrachtung der unterschiedlichen Anordnungen der Reagenzstriche zeigt sich, dass die Auftragevarianten, bei welchen die Probe zuerst mit dem Nachweisreagenz in Kontakt kommt (Kurve A), gegenüber den Auftragevarianten, bei welchen die Probe zuerst mit dem Faktor Xa-Reagenz in Kontakt kommt (Kurven B und C), einen deutlich verzögerten Einsatz der thrombininduzierten Substratspaltung zeigen. Auftragevarianten, bei welchen die Probe zuerst mit dem Faktor Xa-Reagenz und anschließend erst mit dem Nachweisreagenz in Kontakt kommt, sind vor allem aufgrund der rascheren Reaktionskinetiken für die erfindungsgemäße Bestimmung von Faktor Xa-Inhibitoren, insbesondere bei diagnostischen Anwendungen, besonders vorteilhaft.

Ein in Flussrichtung der Probe vor dem Reagenzstrich des Nachweisreagenzes versetzter Auftrag des Reagenzstrichs des Faktor Xa-Reagenzes (Kurve C) zeigt gegenüber dem Auftrag des Reagenzstrichs des Faktor Xa-Reagenzes direkt über dem Reagenzstrich des Nachweisreagenzes (Kurve B) einen geringeren Versatz des Minimums zu höheren Stromwerten sowie einen geringeren Eingangsstrom sowie etwas verlangsamte Reaktionskinetiken.

### Beispiel 4: Einfluss der Anordnungen der Reagenzstriche auf dem Testelement, welches Faktor X-Reagenz und ein Aktivator-Reagenz enthält

Figur 3 zeigt die Ergebnisse elektrochemischer Thrombinbestimmungen bei unterschiedlichen Anordnungen der Reagenzstriche eines Aktivator-Reagenzes und einer Nachweisreagenz/Faktor X-Reagenz-Kombination auf einem Testelement. Dargestellt ist jeweils der zeitliche Verlauf der gemessenen Stromstärke, wobei auf der x-Achse die Zeit t in Sekunden nach der Auftragung der Blutprobe und auf der y-Achse die zum jeweiligen Zeitpunkt bestimmte Stromstärke I in Nanoampere aufgetragen ist. Im vorliegenden Beispiel enthielt der Reagenzstrich der Nachweisreagenz/Faktor X-Reagenz-Kombination 0,8ml reduziertes Chromozyms TH und 38,1 µl Faktor X (factor X der Firma american diagnostica; entsprechend 2U/mI Faktor Xa), der Reagenzstrich des Aktivator-Reagenz 80µl Russels Viper Venom (Russels Viper Venom (RVV-X) der Firma Pentapharm Ltd.) und 0,04% Phospholipide (PHL).

### Die Reagenzstriche wurden in folgenden Varianten aufgetragen:

Kurve A: Zuerst wurde der Reagenzstrich der Nachweisreagenz/Faktor X-Reagenz-Kombination aufgetragen, über diesen dann der Reagenzstrich des Aktivator-Reagenzes.

Kurve B: Zuerst wurde der Reagenzstrich der Nachweisreagenz/Faktor X-Reagenz-Kombination aufgetragen, über diesen dann der Reagenzstrich des Aktivator-Reagenzes, wobei die Auftragung des Reagenzstriches des Aktivator-Reagenzes versetzt erfolgte, so dass dieser zumindest teilweise in Flussrichtung der Probe vor dem Reagenzstrich der Nachweisreagenz/Faktor X-Reagenz-Kombination liegt und diesen zumindest teilweise überdeckt.

Beide Kurven in Figur 3 zeigt keinen Versatz des Minimums zu höheren Stromwerten sowie einen niedrigen Eingangsstrom. Dies deutet darauf hin, dass mit diesen bevorzugten erfindungsgemäßen Verfahren und Vorrichtungen der vorzeitige Substratabbau durch die zumindest teilweise räumliche Trennung der Reagenzien in zwei Kompartimente, insbesondere Reagenzstriche, größtenteils unterbunden werden kann.

Ein in Flussrichtung der Probe vor dem Reagenzstrich der Nachweisreagenz/Faktor X-Reagenz-Kombination versetzter Auftrag des Reagenzstrichs des Aktivator-Reagenz (Kurve B) zeigt gegenüber dem Auftrag des Reagenzstrichs des Aktivator-Reagenzes direkt über dem Reagenzstrich der Nachweisreagenz/Faktor X-Reagenz-Kombination (Kurve A) geringere Strommaxima sowie verlangsamte Reaktionskinetiken. Ein Auftrag des Reagenzstrichs des Aktivator-Reagenz direkt über dem Reagenzstrich der Nachweisreagenz/Faktor X-Reagenz-Kombination sind vor allem aufgrund der rascheren Reaktionskinetiken für die erfindungsgemäße Bestimmung von Faktor Xa-Inhibitoren, insbesondere bei diagnostischen Anwendungen, besonders vorteilhaft.

### Beispiel 5: Bestimmung unterschiedlicher Faktor Xa-Inhibitor-Konzentration

Im vorliegenden Beispiel wurden Vollblutproben vermessen, denen unterschiedliche Mengen des Faktor Xa-Inhibitors Enoxaparin-Natrium (Clexane^{®}, Aventis) zugesetzt wurden. Zur Bestimmung dieses Faktor Xa-Inhibitors wurden erfindungsgemäße Testelemente entsprechend Beispiel 3 eingesetzt, wobei der Reagenzstrich des Faktor Xa-Reagenzes jeweils 2U/mI Faktor Xa und zusätzlich 0,04 % Phospholipide (PHL), der Reagenzstrich des Nachweisreagenzes 0,8 ml reduziertes Chromozyms TH enthielt. Hierbei wurde zuerst der Reagenzstrich des Nachweisreagenzes aufgetragen, dann der Reagenzstrich des Faktor Xa-Reagenzes, wobei die Auftragung des Reagenzstriches des Faktor Xa-Reagenzes versetzt erfolgte, so dass dieser zumindest teilweise in Flussrichtung der Probe vor dem Reagenzstrich des Nachweisreagenzes liegt und diesen zumindest teilweise überdeckt.

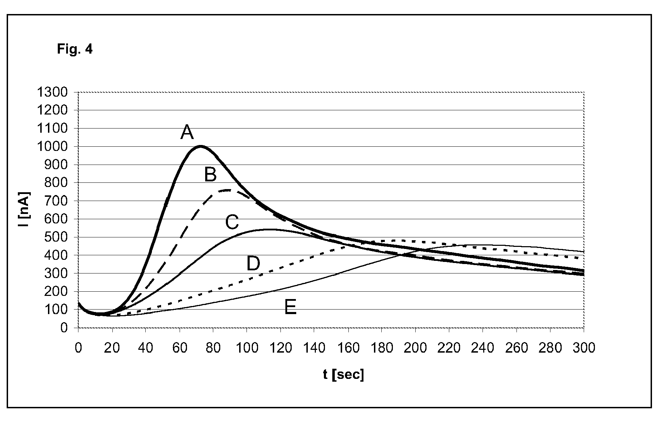

Figur 4 zeigt die Ergebnisse elektrochemischer Faktor Xa-Inhibitor-Bestimmungen von Vollblutproben unterschiedlicher Faktor Xa-Inhibitor-Konzentration. Dargestellt ist jeweils der zeitliche Verlauf der gemessenen Stromstärke, wobei auf der x-Achse die Zeit t in Sekunden nach der Auftragung der Blutprobe und auf der y-Achse die zum jeweiligen Zeitpunkt bestimmte Stromstärke I in Nanoampere aufgetragen ist. Kurve A zeigt ein exemplarisches Messergebnis einer Probe mit 0 U/ml Enoxaparin, Kurve B zeigt ein exemplarisches Messergebnis einer Probe mit 0,25 U/ml Enoxaparin, Kurve C zeigt ein exemplarisches Messergebnis einer Probe mit 0,5 U/ml Enoxaparin, Kurve D zeigt ein exemplarisches Messergebnis einer Probe mit 0,75 U/ml Enoxaparin und Kurve A zeigt ein exemplarisches Messergebnis einer Probe mit 1,0 U/ml Enoxaparin.

Dieses Beispiel zeigt deutlich, dass mit den erfindungsgemäßen Vorrichtungen und Verfahren verschiedene in Vollblutproben vorliegende Konzentrationen von Faktor Xa-Inhibitoren klar voneinander unterschieden werden können. Betrachtet man den Verlauf der Kurven, zeigt sich, dass mit zunehmender Konzentration des Faktor Xa-Inhibitors in der Probe der Kurvenverlauf immer flacher wird, was einer verzögerten Bildung der Spaltprodukte durch die Thrombin-induzierte Spaltung entspricht. Während die Probe in Kurve A keinen Faktor Xa-Inhibitor enthält und der Kurvenverlauf dort sehr rasch zu einem hohen Stromstärkenmaximum ansteigt, zeigt die Kurve 5, welche einer Probe mit einem hohen Faktor Xa-Inhibitorgehalt entspricht, dagegen einen deutlich verzögerten Kurvenanstieg und ein deutlich geringeres Stromstärkenmaximum. Die einzelnen Konzentrationen des Faktor Xa-Inhibitors können mit diesen Testelementen sehr regelmäßig in ihren zeitlichem Verlauf der Thrombinumsetzung abgestuft werden. In allen Fällen war durch die erfindungsgemäße Anordnung der Reagenzien kein Versatz des Minimums zu höheren Eingangsstromstärken und somit kein vorzeitiger Substratabbau zu beobachten. Eine Auswertung der Kurven mit einem Schwellwertalgorithmus, wie er zuvor beschrieben wurde, führte zu folgenden zeitlichen Messgrößen für die Thrombinumsetzung, welche mit den entsprechenden Faktor Xa-Inhibitor-Konzentrationen korrelieren:

| **Enoxaparin [U/ml]** | **Zeitparameter für die Thrombinumsetzung [sec]** |
|---|---|
| 0 | 33,35 |
| 0,25 | 36,83 |
| 0,5 | 40,72 |
| 0,75 | 59,86 |
| 1 | 80,32 |

Anhand solcher Faktor Xa-Inhibitor-Bestimmungen mit Proben bekannter Konzentrationen an Faktor Xa-Inhibitor können beispielsweise Beziehungen zwischen den gemessen Stromverläufen bzw. den davon abgeleiteten Zeitparametern und den jeweiligen Faktor Xa-Inhibitor-Konzentrationen bestimmt werden, beispielsweise in Form von Eichkurven. Anhand solcher Beziehungen kann dann aus den bestimmten Stromverläufen bzw. den davon abgeleiteten Zeitparametern einer Probe unbekannter Faktor Xa-Inhibitor-Konzentration auf die Konzentrationen dieses Faktor Xa-Inhibitors in dieser Probe geschlossen werden.

## Patentansprüche

1. Verfahren zur Bestimmung von Faktor Xa-Inhibitoren in Blutproben,
**dadurch gekennzeichnet, dass**
a) eine Probe des zu untersuchenden Blutes mit einem Nachweisreagenz, welches zumindest ein Thrombinsubstrat, welches aus einem Peptidrest besteht, der durch Thrombin abgespalten werden kann und der über das Carboxylende amidisch an eine elektrogene Substanz gebunden ist, enthält, und mit einer bekannten Menge von Faktor Xa in Kontakt gebracht wird,
b) die Menge oder Aktivität der elektrogenen Substanz, welche durch die Faktor Xa-vermittelte Thrombinaktivierung vom Thrombinsubtrat abgespalten wird, und/oder deren zeitlicher Verlauf als Messsignal mit elektrochemischen Methoden bestimmt wird, und
c) anhand dieses Messsignals die Menge des Faktor Xa-Inhibitors in der Probe des zu untersuchenden Blutes oder eine damit korrelierende Messgröße, insbesondere eine damit korrelierende Gerinnungszeit, ermittelt wird.

2. Verfahren gemäß Anspruch 1,
**dadurch gekennzeichnet, dass**
eine bekannte Menge von Faktor Xa-Reagenz in Schritt a) der Probe zugesetzt wird

3. Verfahren gemäß Anspruch 2,
**dadurch gekennzeichnet, dass**
der Zusatz von Faktor Xa-Reagenz zur Probe räumlich oder zeitlich getrennt vom Zusatz des Nachweisreagenz zur Probe erfolgt.

4. Verfahren gemäß einem der Ansprüche 2 und 3,
**dadurch gekennzeichnet, dass**
der Zusatz von Faktor Xa-Reagenz zur Probe vor dem Zusatz des Nachweisreagenz zur Probe erfolgt.

5. Verfahren gemäß Anspruch 1,
**dadurch gekennzeichnet, dass**
eine bekannte Menge von Faktor Xa in Schritt a) durch Zugabe einer bekannten Menge von Faktor X-Reagenz und eines Aktivator-Reagenz, welche die Umwandlung von Faktor X in Faktor Xa hervorruft, zur Probe erreicht wird.

6. Verfahren gemäß Anspruch 5,
**dadurch gekennzeichnet, dass**
als Aktivator-Reagenz ein Aktivator oder ein beteiligter Komplex des extrinsischen Wegs der plasmatischen Gerinnung, insbesondere Tissue Factor und/oder Faktor VIIa, oder ein Aktivator oder ein beteiligter Komplex des intrinsischen Wegs der plasmatischen Gerinnung, insbesondere Faktor XIIa,oder eine Substanz, welche eine Umwandlung von Faktor X in Faktor Xa hervorruft, insbesondere Russels Viper Venom, eingesetzt wird.

7. Verfahren gemäß Anspruch 5 oder 6,
**dadurch gekennzeichnet, dass**
der Zusatz von Faktor X-Reagenz zur Probe räumlich oder zeitlich getrennt vom Zusatz des Aktivator-Reagenz zur Probe erfolgt.

8. Verfahren gemäß Anspruch 7,
**dadurch gekennzeichnet, dass**
das Faktor X-Reagenz ein Bestandteil des Nachweisreagenz ist und der Zusatz dieser Nachweisreagenz/Faktor X-Reagenz-Kombination zur Probe räumlich oder zeitlich getrennt vom Zusatz des Aktivator-Reagenz zur Probe, insbesondere nach dem Zusatz des Aktivator-Reagenz zur Probe, erfolgt.

9. Verfahren gemäß Anspruch 5 oder 6,
**dadurch gekennzeichnet, dass**
der Zusatz von Faktor X zur Probe in einem gemeinsamen Schritt mit dem Zusatz des Aktivators zur Probe erfolgt.

10. Verfahren gemäß Anspruch 9,
**dadurch gekennzeichnet, dass**
Faktor X-Reagenz und Aktivator-Reagenz gemeinsam in einem trockenen Zustand vorliegen und die Umwandlung von Faktor X in Faktor Xa erst durch den Kontakt mit der Probe bewirkt wird.

11. Verfahren gemäß Anspruch 10,
**dadurch gekennzeichnet, dass**
Faktor X-Reagenz, Aktivator-Reagenz und Nachweisreagenz gemeinsam in einem trockenen Zustand vorliegen und die Umwandlung von Faktor X in Faktor Xa erst durch den Kontakt mit der Probe bewirkt wird.

12. Verfahren gemäß einem der vorherigen Ansprüche,
**dadurch gekennzeichnet, dass**
der Faktor Xa-Inhibitor ein Heparin, insbesondere ein fraktioniertes oder niedermolekulares Heparin, ein indirekter selektiver oder ein direkter Faktor Xa-Inhibitor ist.

13. Testelement mit einem elektrochemischen Sensor auf trockenchemischer Basis zur Bestimmung von Faktor Xa-Inhibitoren, insbesondere unfraktionierten Heparinen und fraktionierten oder niedermolekularen Heparinen, indirekten selektiven Faktor Xa-Inhibitoren und direkten Faktor Xa-Inhibitoren, der auf einem inerten Träger mindestens zwei Elektroden aufweist und ein Nachweisreagenz, welches zumindest ein Thrombinsubstrat, welches aus einem Peptidrest besteht, der durch Thrombin abgespalten werden kann und der über das Carboxylende amidisch an eine elektrogene Substanz gebunden ist, sowie zumindest eine bestimmte Menge von Faktor X-Reagenz und ein Aktivator-Reagenz oder eine bestimmte Menge von Faktor Xa-Reagenz enthält.

14. Testelement gemäß Anspruch 13,
**dadurch gekennzeichnet, dass**
es Faktor Xa-Reagenz enthält und das Faktor Xa-Reagenz auf dem Testelement zumindest teilweise räumlich getrennt vom Nachweisreagenz vorliegt, insbesondere zumindest teilweise in Flussrichtung der Probe vor dem Nachweisreagenz angeordnet ist.

15. Testelement gemäß Anspruch 13,
**dadurch gekennzeichnet, dass**
es Faktor X-Reagenz und ein Aktivator-Reagenz, welches die Umwandlung von Faktor X in Faktor Xa hervorruft, enthält und das Aktivator-Reagenz auf dem Testelement zumindest teilweise räumlich getrennt vom Faktor X-Reagenz vorliegt, insbesondere zumindest teilweise in Flussrichtung der Probe vor dem Faktor X-Reagenz angeordnet ist.

16. Testelement gemäß Anspruch 15,
**dadurch gekennzeichnet, dass**
das Nachweisreagenz gemeinsam mit dem Aktivator-Reagenz oder dem Faktor X-Reagenz auf dem Testelement vorliegt.

17. Testelement gemäß Anspruch 13,
**dadurch gekennzeichnet, dass**
es Faktor X-Reagenz und ein Aktivator-Reagenz, welches die Umwandlung von Faktor X in Faktor Xa hervorruft, als trockenchemische Reagenzien enthält und das Aktivator-Reagenz auf dem Testelement gemeinsam mit dem Faktor X-Reagenz, optional auch gemeinsam mit dem Nachweisreagenz, vorliegt, so dass die Umwandlung von Faktor X in Faktor Xa erst durch den Kontakt mit der Probe bewirkt wird.

18. Elektrochemisches Testelement-Analysesystem,
enthaltend
zumindest ein Strommessgerät und ein Testelement gemäß einem der Ansprüche 13 bis 17.
